(19) Europäisches Patentamt
European Patent Office
Office européen des brevets

(11) Veröffentlichungsnummer: **0 284 702**
**B1**

(12) # EUROPÄISCHE PATENTSCHRIFT

(45) Veröffentlichungstag der Patentschrift: **08.11.89**

(51) Int. Cl.⁴: **C07D 249/08**
// C07C49/39

(21) Anmeldenummer: **87810204.5**

(22) Anmeldetag: **03.04.87**

(54) **Substituiertes Cyclobutanon und Verfahren zu seiner Herstellung.**

(43) Veröffentlichungstag der Anmeldung:
**05.10.88 Patentblatt 88/40**

(45) Bekanntmachung des Hinweises auf die Patenterteilung:
**08.11.89 Patentblatt 89/45**

(84) Benannte Vertragsstaaten:
**DE**

(56) Entgegenhaltungen:
**EP-A- 0 152 031**
**WO-A-85/05358**

(73) Patentinhaber: **CIBA-GEIGY AG, Klybeckstrasse 141, CH-4002 Basel(CH)**

(72) Erfinder: **Martin, Pierre, Dr., Meisenweg 38, CH-4310 Rheinfelden(CH)**

**EP 0 284 702 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

In der WO 85/05358 sind einen Triazolylrest enthaltende Cycloalkanone beschrieben. Der Triazolylrest ist über ein gegebenenfalls substituiertes C-Atom an das Cycloalkanon gebunden.

Gegenstand der Erfindung ist 2-(p-Chlorphenoxy)-2-triazolo-3,3-dimethylcyclobutan-1-on der Formel

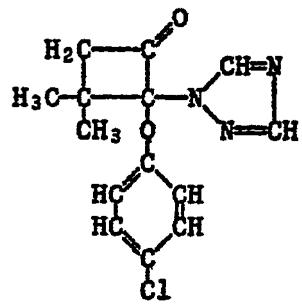

Es handelt sich um eine Verbindung mit öliger Konsistenz, die hergestellt werden kann, indem man

a) 2,4-Dichlor-3,3-dimethylcyclobutan-1-on, 2,2-Dichlor-3,3-dimethylcyclobutan-1-on oder Mischungen hiervon mit einer alkalischen Lösung von p-Chlorphenol umsetzt, das Reaktionsprodukt isoliert und danach

b) das Reaktionsprodukt mit einem Alkalisalz des Triazols umsetzt.

2,4-Dichlor- bzw. 2,2-Dichlor-3,3-dimethylcyclobutan-1-on sind in Helvetia Chimica Acta 64, 2571 (1981) beschrieben.

Die Reaktionsstufe a) wird zweckmässig bei einer Temperatur von 0 bis 30°C und in Gegenwart eines inerten Lösungsmittels durchgeführt. Geeignete Lösungsmittel sind halogenierte Kohlenwasserstoffe, wie z.B. Methylenchlorid, Chloroform, Tetrachlorkohlenstoff, 1,1,2-Trichlorethan oder 1,1,2,2-Tetrachlorethan.

Als alkalische Lösungen kommen z.B. wässrige Kalilauge und besonders Natronlauge in Frage. Die Konzentration an Alkalihydroxid kann 5-30 Gew.-% betragen. Zweckmässig werden solche Mengen Alkalilauge verwendet, dass das p-Chlorphenol als Alkalisalz vorliegt.

Die Isolierung des Reaktionsproduktes erfolgt vorteilhaft durch Extraktion mit einem mit Wasser wenig oder nicht mischbaren Lösungsmittel und anschliessendes Verdampfen des Lösungsmittels. Besonders geeignete Lösungsmittel sind Kohlenwasserstoffe, wie z.B. Pentan, Hexan, Cyclohexan, Methylcyclohexan und Petrolether, oder Ether, wie z.B. Diethylether, Tetrahydrofuran oder Dioxan. Das isolierte Reaktionsprodukt kann direkt weiterverwendet werden. Es kann aber auch zuvor, z.B. mittels chromatographischer Methoden, gereinigt werden. Die Reaktanden der Reaktionsstufe a) werden zweckmässig im molaren Verhältnis von 1:1 eingesetzt. Es kann aber auch ein geringer Ueberschuss an p-Chlorphenol verwendet werden.

Die Umsetzung der Reaktionsstufe b) wird unter Inertgas, z.B. Stickstoff oder Argon, durchgeführt. Vorteilhaft wird ein inertes Lösungsmittel mitverwendet, z.B. N-alkylierte Säureamide oder Lactame. Beispiele sind Dimethylformamid (DMF), Dimethylacetamid und N-Methylpyrrolidon. Die Reaktionstemperatur beträgt vorteilhaft 50 bis 150°C. Als Alkalisalz des Triazols kommt insbesondere das Natriumsalz in Frage. Die Reaktanden der Reaktionsstufe b) werden bevorzugt im molaren Verhältnis von 1:1 eingesetzt. Man kann auch einen geringeren Ueberschuss des Alkalisalzes des Triazols verwenden.

Die Isolierung des 2-(p-Chlorphenoxy)-2-triazolo-3,3-dimethylcyclobutan-1-ons erfolgt in an sich bekannter Weise durch Extraktion mit z.B. Diethylether, Waschen der Etherlösung, Trocknen der Etherlösung und Verdampfen des Ethers. Der ölige Rückstand kann durch Chromatographie weiter gereinigt werden.

Beispiel:

a) 2-Chlor-4-(p-chlorphenoxy)-3,3-dimethylcyclobutan-1-on

Zu 41,8 g eines 4:1-Gemisches von 2,4-Dichlor-3,3-dimethylcyclobutan-1-on und 2,2-Dichlor-3,3-dimethylcyclobutan-1-on [Helv.Chim. Acta 64, 2571 (1981)], gelöst in 100 ml Chloroform, werden 5,0 g Tetrabutylammoniumchlorid gegeben und die Mischung auf 5°C gekühlt. Dazu wird während 55 Minuten eine Lösung von 32,1 g p-Chlorphenol in 100 ml NaOH (10 %ig) getropft und 15 Minuten bei 5°C nachgerührt. Das Reaktionsgemisch wird mit Ether mehrmals ausgezogen, die organische Phase mit Wasser gewaschen, getrocknet ($MgSO_4$) und eingedampft. Der Rückstand (73,9 g) wird über Kieselgel chromatographiert (Toluol/Hexan 1:1).

Man erhält 59,8 g Produkt als sprödes Harz.

$^1$H-NMR ($CDCl_3$): 1,20 (s, 3H, $CH_3$);
1,70 (s, 3H, $CH_3$);
4,55 (d, J = 3 Hz, 1H, CH);
4,95 (d, J = 3 Hz, 1H, CH);
7,1 (AB-System, J = 8 Hz, 4H, aromatische H).

b) 2-(p-Chlorphenoxy)-2-triazolo-3,3-dimethylcyclobutan-1-on

Unter Stickstoff werden 6,9 g Triazol in 70 ml DMF eingetragen und auf 80°C erwärmt. Dazu werden portionenweise 4,4 g NaH-Dispersion (55 %ig) zugegeben und 2 Stunden bei 80°C gerührt. Dann werden 25,9 g des oben beschriebenen Reaktionsproduktes, gelöst in 200 ml DMF, bei 80°C zugetropft und 1 Stunde bei dieser Temperatur nachgerührt und danach abgekült. Das Gemisch wird auf 1 kg Eis gegossen und mehrmals mit Ether extrahiert. Die vereinigten Etherphasen werden mit Sole und Wasser gewaschen, getrocknet ($MgSO_4$) und eingedampft. Das Rohöl (24,5 g) wird über Kieselgel chromatographiert (Toluol/Essigester 4:1).

Man erhält die Titelverbindung als zähes Oel.

$^1$H-NMR ($CDCl_3$); 1,05 (s, 3H, $CH_3$);
1,70 (s, 3H, $CH_3$);

3,15 (AB-System, J = 18 Hz, 2H, $CH_2$);
6,95 (AB-System, J = 8 Hz, 4H, aromatische H);
8,05 und 8,25 (je s, je 1H, Triazolo-H).

**Patentansprüche**

1. 2-(p-Chlorphenoxy)-2-triazolo-3,3-dimethylcyclobutan-1-on der Formel

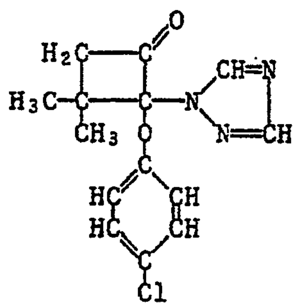

2. Verfahren zur Herstellung von 2-(p-Chlorphenoxy)-2-triazolo-3,3-dimethylcyclobutan-1-on der Formel

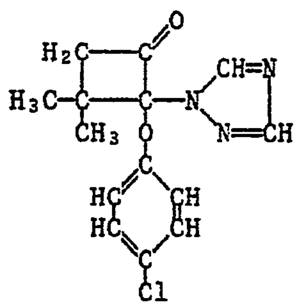

dadurch gekennzeichnet, dass man
a) 2,4-Dichlor-3,3-dimethylcyclobutan-1-on, 2,2-Dichlor-3,3-dimethylcyclobutan-1-on oder Mischungen hiervon mit einer alkalischen Lösung von p-Chlorphenol umsetzt, das Reaktionsprodukt isoliert und danach
b) das Reaktionsprodukt mit einem Alkalisalz des Triazols umsetzt.

**Claims**

1. A 2-(p-chlorophenoxy)-2-triazolo-3,3-dimethylcyclobutan-1-one of formula

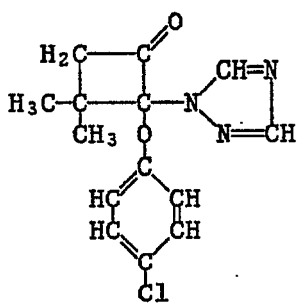

2. A process for the preparation of a 2-(p-chlorophenoxy)-2-triazolo-3,3-dimethylcyclobutan-1-one of formula

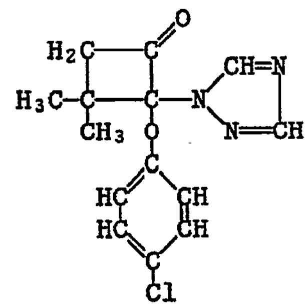

which comprises
a) a reacting 2,4-dichloro-3,3-dimethylcyclobutan-1-one, 2,2-dichloro-3,3-dimethylcyclobutan-1-one, or a mixture thereof, with an alkaline solution of p-chlorophenol, isolating the reaction product, and subsequently
b) reacting said reacting product with an alkali metal salt of the triazole.

**Revendications**

1. 2-(p-Chlorphenoxy)-2-triazolo-3,3-diméthylcyclobutanone-1 de formule

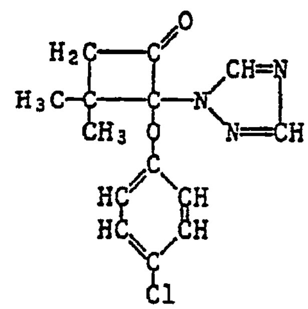

2. Procédé de préparation de 2-(p-chlorphenoxy)-2-triazolo-3,3-diméthylcyclobutanone-1 de formule

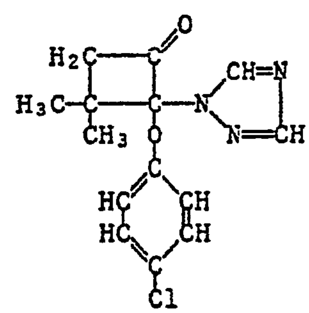

caractérisé en ce que l'on fait réagir
a) 2,4-dichloro-3,3-diméthylcyclobutanone-1, 2,2-dichloro-3,3-diméthylcyclobutanone-1 ou des mélanges de ces deux produits, avec une solution alcaline de p-chlorophenol, que l'on isole le produit de réaction et qu'ensuite
b) l'on fait réagir le produit de réaction avec un sel alcaline du triazole.